# EUROPEAN PATENT APPLICATION

(11) **EP 4 094 749 A1**
(43) Date of publication of application: **30.11.2022**
(21) Application number: 20915198.4
(22) Date of filing: 11.11.2020
(51) Int. Cl.: A61K 8/00, A61K 8/55, A61K 8/64, A61K 47/24, A61K 47/42

(54) **THICKENER**

(30) Priority: 22.01.2020 JP 2020008230
(71) Applicant: National Institute Of Advanced Industrial Science and Technology, Chiyoda-ku Tokyo 100-8921 (JP); KANEKA CORPORATION, Osaka 530-8288 (JP)
(72) Inventor: IMURA, Tomohiro, Tsukuba-shi, Ibaraki 305-8560 (JP); TAIRA, Toshiaki, Tsukuba-shi, Ibaraki 305-8560 (JP); TSUJI, Tadao, Settsu-shi, Osaka 566-0072 (JP); YANAGISAWA, Satohiro, Tokyo 107-6028 (JP)
(74) Representative: Hoffmann Eitle
(86) International application number: PCT/JP2020/042040
(87) International publication number: WO 2021/149334

(57) **Abstract**

The objective of the present invention is to provide a thickener that can increase the viscosity of a composition with suppressing stickiness and maintaining use feeling in the case of applying the composition on the skin or the like, since the thickener does not contain a polymer thickener or the content amount of a polymer thickener is small. The thickener of the present invention is characterized in comprising a phospholipid and a biosurfactant, wherein a ratio of the phospholipid to 1 part by mass of the biosurfactant is more than 4 parts by mass and 10 parts or less by mass.

## Description

### TECHNICAL FIELD

The present invention relates to a thickener that can increase the viscosity of a composition while suppressing stickiness and maintaining a good feeling when the composition is applied on the skin or the like, since the thickener does not contain a polymer thickener or the amount of a polymer thickener is small.

### BACKGROUND ART

A topical medication to be applied on the skin or the like becomes easily applied while suppressing dripping by increasing the viscosity as compared with a liquid formulation, such as an aqueous solution and a suspension. A thickener is therefore mixed in a topical medication to increase viscosity in some cases.

An example of a thickener generally includes a polymer such as a protein and a polysaccharide. An example of a protein thickener includes gelatin. An example of a polysaccharide thickener includes xanthane gum derived from a microorganism, carrageenan derived from seaweed, and guar gum and a cellulose derivative derived from a plant. On the one hand, a polymer thickener causes stickiness and impairs the good feeling upon use, instead feeling like an adhered syrup, especially after a topical medication is applied and dried. Thus, a compound and a composition that are low molecular weight and have a thickening action are preferred, but a practical low molecular weight thickener has not ever been developed.

For example, Patent document 1 discloses a block copolymer having a hydrophobic polymer unit and a sulfonate group-containing hydrophilic polymer unit as a thickener component having a relatively low molecule weight. However, the above-described problem of stickiness cannot be solved by the block copolymer, since the molecular weight of the block copolymer is several tens of thousands or more.

### PRIOR ART DOCUMENT

### PATENT DOCUMENT

Patent document 1: JP H11-241060 A

### DISCLOSURE OF THE INVENTION

### PROBLEMS TO BE SOLVED BY THE INVENTION

A conventional thickener has a problem of stickiness, since a conventional thickener is a polymer such as a protein and a polysaccharide, as described above.

Accordingly, the objective of the present invention is to provide a thickener that can increase the viscosity of a composition while suppressing stickiness and maintaining good feeling when applied on the skin or the like, since the thickener does not contain a polymer thickener or the amount of a polymer thickener is small.

### MEANS FOR SOLVING THE PROBLEMS

The inventors of the present invention made extensive studies to solve the above-described problem. As a result, the inventors completed the present invention by finding that a molecular aggregate composed of threads entangled with each other was formed and a thickener can be obtained without using a polymer thickener by using a phospholipid and a biosurfactant and appropriately adjusting the ratios thereof.

Hereinafter, the present invention is described.
[1] A thickener,
   comprising a phospholipid and a biosurfactant,
   wherein a ratio of the phospholipid to 1 part by mass of the biosurfactant is more than 4 parts by mass and 10 parts or less by mass.
[2] The thickener according to the above [1], comprising a thread-like molecular aggregate comprising the phospholipid and the biosurfactant.
[3] The thickener according to the above [2], comprising 1 part or more by mass and 20 parts or less by mass of a polyol to 1 part by mass of the biosurfactant.
[4] The thickener according to any one of the above [1] to [3], wherein the biosurfactant is surfactin represented by the following formula (II) or a salt thereof: 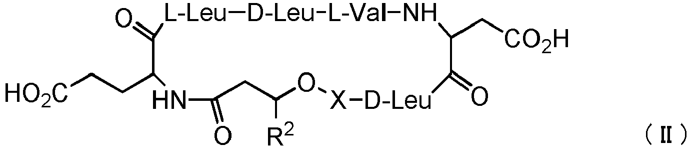 wherein X is an amino acid residue selected from leucine, isoleucine and valine, and R² is a C₉₋₁₈ alkyl group.
[5] A topical medication comprising the thickener according to any one of the above [1] to [4].
[6] The topical medication according to the above [5], wherein a concentration of a polymer thickener in the composition is less than 1.0 mass%.
[7] The topical medication according to the above [5] or [6], wherein the topical medication is a cosmetic.
[8] Use of a thickener for increasing viscosity of a composition comprising water, wherein the composition comprises a phospholipid and a biosurfactant, and a ratio of the phospholipid to 1 part by mass of the biosurfactant is more than 4 parts by mass and 10 parts or less by mass.
[9] The use according to the above [8], wherein the thickener comprises a thread-like molecular aggregate comprising the phospholipid and the biosurfactant.
[10] The use according to the above [8] or [9], wherein the thickener comprises 1 mass part or more by mass and 20 parts or less by mass of a polyol to 1 part by mass of the biosurfactant.
[11] The use according to any one of the above [8] to [10], wherein the biosurfactant is surfactin represented by the formula (II) or a salt thereof.
[12] The use according to any one of the above [8] to [11], wherein a concentration of a polymer thickener in the composition is less than 1.0 mass%.
[13] A method for increasing viscosity of a composition comprising water,
   comprising the step of mixing a thickener in the composition,
   wherein the thickener comprises a phospholipid and a biosurfactant, and a ratio of the phospholipid to 1 part by mass of the biosurfactant is more than 4 parts by mass and 10 parts or less by mass.
[14] The method according to the above [13], wherein the thickener comprises a thread-like molecular aggregate comprising the phospholipid and the biosurfactant.
[15] The method according to the above [13] or [14], wherein the thickener comprises 1 mass part or more by mass and 20 parts or less by mass of a polyol to 1 part by mass of the biosurfactant.
[16] The method according to any one of the above [13] to [15], wherein the biosurfactant is surfactin represented by the formula (II) or a salt thereof.
[17] The method according to any one of the above [13] to [16], wherein a concentration of a polymer thickener in the composition is less than 1.0 mass%.

### EFFECT OF THE INVENTION

The thickener of the present invention can increase the viscosity of a composition even when a polymer thickener is not used or the amount thereof is reduced. As a result, when a composition containing the thickener of the present invention is applied to the skin or the like, the stickiness caused by a polymer thickener is suppressed particularly after the composition is dried. Thus, the thickener of the present invention is very useful as a component to increase the viscosity of a topical medication, such as a cosmetic.

### BRIEF DESCRIPTION OF THE DRAWINGS

Figure 1 are photographs of the thickener according to the present invention.
Figure 2 are graphs to demonstrate the measurement result of dynamic viscoelasticity of the thickener according to the present invention.
Figure 3 is a photograph to demonstrate the magnification observation result of the thickener according to the present invention using a freeze-fracture transmission electron microscope.

### MODE FOR CARRYING OUT THE INVENTION

The thickener of the present invention exhibits a thickening property, even though the thickener does not contain a polymer thickener or the amount of a polymer thickener is less than 1.0 mass%.

The molecular weight of a polymer thickener is not particularly restricted in this disclosure as long as the polymer thickener can be said to be a polymer, and for example, may be 10,000 or more. An example of a general polymer thickener includes a cellulose polymer thickener such as cellulose and a derivative thereof; an alginate polymer thickener such as sodium alginate; a starch polymer thickener such as starch, carboxymethyl starch and methyl hydroxypropyl starch; other polysaccharide polymer thickener such as agar, xanthane gum, carrageenan and guar gum; a protein polymer thickener such as pectin, collagen, casein, albumin and gelatin; a vinyl polymer thickener such as polyvinyl methyl ether and carboxy vinyl polymer; a polyoxyethylene polymer; a polyoxyethylene-polyoxypropylene copolymer; and an acrylate polymer thickener such as polyethyl acrylate and polyacrylamide.

A phospholipid is a general term for a lipid having a phosphate ester part in the structure and classified into a glycerophospholipid having a glycerin in the skeleton and a sphingophospholipid having a sphingosine in the skeleton. A sphingosine has a structure obtained by replacing the hydroxy group at the C2 position of glycerin with an amino group and further binding a long chain alkyl group at the C1 position. The phosphate group forms a phosphate ester with the hydroxy group of the glycerin or the sphingosine. A gelatinous composition and an emulsified composition containing a phospholipid are excellent in feeling when used, since a phospholipid is one of the constituent components of a cell membrane.

For example, the phospholipid is represented by the following general formula (III): 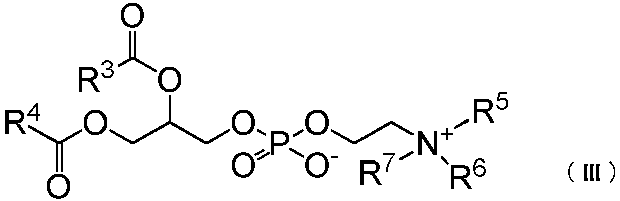 wherein R³ and R⁴ are independently a C₈₋₂₄ alkyl group, a C₈₋₂₄ alkenyl group or a C₈₋₂₄ alkynyl group, and R⁵ to R⁷ are independently a C₁₋₆ alkyl group.

The C₈₋₂₄ alkyl group means a linear or branched monovalent saturated aliphatic hydrocarbon group having a carbon number of 8 or more and 24 or less. An example of the group includes octyl, nonyl, decyl, dodecyl, tetradecyl, hexadecyl, octadecyl, eicosyl, docosyl, isotetradecyl, isohexadecyl, isooctadecyl, isoeicosyl, isodocosyl, 2-butyldecyl, 2-hexyldecyl, 2-octyldecyl, 2-decanyldecyl, 2-dodecanyldecyl and tetracosyl.

The C₈₋₂₄ alkenyl group means a linear or branched monovalent unsaturated aliphatic hydrocarbon group having a carbon number of 8 or more and 24 or less and having at least one carbon-carbon double bond.

The C₈₋₂₄ alkynyl group means a linear or branched monovalent unsaturated aliphatic hydrocarbon group having a carbon number of 8 or more and 24 or less and having at least one carbon-carbon triple bond.

The C₁₋₆ alkyl group means a linear or branched monovalent saturated aliphatic hydrocarbon group having a carbon number of 1 or more and 6 or less. An example of the group includes methyl, ethyl, n-propyl, isopropyl, n-butyl, isobutyl, s-butyl, t-butyl, n-pentyl and n-hexyl. The group is preferably a C₁₋₄ alkyl group, more preferably a C₁₋₂ alkyl group, and even more preferably methyl.

A specific example of the phospholipid includes lecithin. Lecithin has the following structure and is another name for phosphatidylcholine.

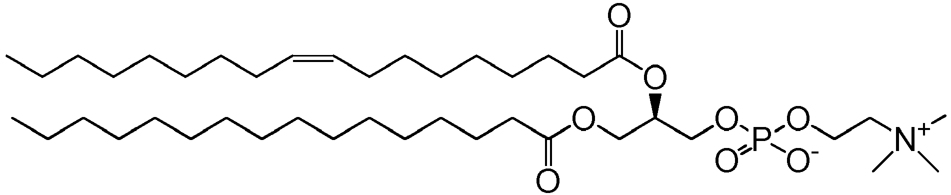

A lipid product containing a naturally derived phospholipid is sometimes called as natural lecithin. For example, an egg-yolk-derived phospholipid product is sometimes called egg-yolk lecithin, and a soybean-derived phospholipid product is sometimes called soybean lecithin.

An example of the phospholipid also includes a glycerophospholipid such as phosphatidic acid, bisphosphatidic acid, phosphatidylserine, phosphatidylethanolamine, phosphatidylmethylethanolamine, phosphatidylinositol, phosphatidylglycerol and diphosphatidylglycerol; a sphingophospholipid such as sphingosine, ceramide, sphingomyelin and cerebroside; and a hydrogenated lecithin such as hydrogenated soybean lecithin and hydrogenated egg-yolk lecithin.

A biosurfactant is a naturally derived surfactant compound produced by a microorganism or the like and exemplified by a lipopeptide biosurfactant having a hydrophobic group and a hydrophilic peptide part, especially a cyclic lipopeptide biosurfactant having a cyclic peptide part. Such a cyclic peptide part has one or more anionic groups such as a carboxy group and a phenolic hydroxy group. The lipopeptide biosurfactant has the advantage of having a small impact on the environment, since the lipopeptide biosurfactant is easily decomposed by a microorganism or the like.

The cyclic lipopeptide biosurfactant is exemplified by one or more cyclic lipopeptide biosurfactants selected from surfactin, arthrofactin, iturin, and salts thereof, and is preferably surfactin or a salt thereof.

The surfactin or a salt thereof is a surfactin represented by the general formula (II) or a salt thereof. 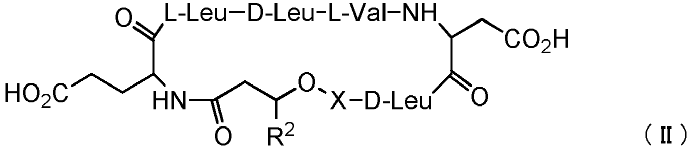 wherein R² and X are the same as the above.

The amino acid residue X may be any one of an L-body and D-body, and is preferably an L-body. An example of the counter cation that constitutes the salt of surfactin includes an alkali metal ion and a quaternary ammonium ion. The alkali metal ion is not particularly restricted, exemplified by a lithium ion, a sodium ion and a potassium ion, and preferably a sodium ion. An example of the substituent group of the quaternary ammonium ion includes an organic group. An example of such an organic group includes a C₁₋₆ alkyl group such as methyl, ethyl, n-propyl, isopropyl, n-butyl and tert-butyl; a C₁₋₆ alkyl group having a substituent group, such as 2-hydroxyethyl; an aralkyl group such as benzyl, methylbenzyl and phenylethyl; and an aryl group such as phenyl, toluyl and xylyl. An example of the quaternary ammonium ion includes a tetramethylammonium ion, a tetraethylammonium and a pyridinium ion.

The C₉₋₁₈ alkyl group means a linear or branched monovalent saturated aliphatic hydrocarbon group having a carbon number of 9 or more and 18 or less. An example of the group includes n-nonyl, 6-methyloctyl, 7-methyloctyl, n-decyl, 8-methylnonyl, n-undecyl, 9-methyldecyl, n-dodecyl, 10-methylundecyl, n-tridecyl, 11-methyldodecyl, n-tetradecyl, n-pentadecyl, n-hexadecyl, n-heptadecyl and n-octadecyl.

Arthrofactin is represented by the general formula (IV).

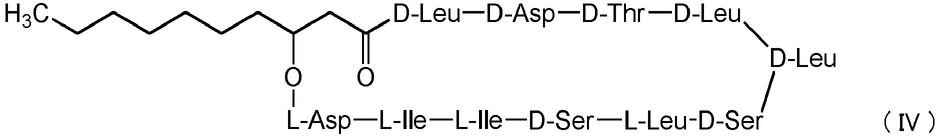

Arthrofactin has one D-aspartic acid and one L-aspartic acid respectively in the structure, and the amino acid residues may form a salt. An example of the counter cation that constitutes such a salt includes an alkali metal ion and a quaternary ammonium ion.

Iturin is represented by the general formula (V).

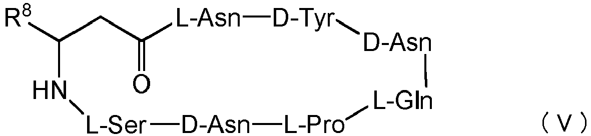

In the formula (V), R⁸ is a C₉₋₁₈ alkyl group and exemplified by -(CH₂)₁₀CH₃, -(CH₂)₈CH(CH₃)CH₂CH₃ and -(CH₂)₉CH(CH₃)₂.

One of the biosurfactant or salt thereof may be used, or two or more of the biosurfactants or salts thereof may be used. For example, the biosurfactant or salt thereof may be a mixture containing two or more of the biosurfactants or salts thereof having hydrophobic groups having different carbon numbers. The cyclic lipopeptide biosurfactant can be purified from a culture liquid in which a microorganism that produces the target cyclic lipopeptide biosurfactant is cultivated in accordance with a publicly known method. A purified product may be used or an unpurified product may be used as the cyclic lipopeptide biosurfactant. For example, the above culture liquid may be directly used. An example of the microorganism that produces surfactin includes a strain classified in Bacillus subtilis. A chemically synthesized biosurfactant may be similarly used.

The phospholipid and the biosurfactant may form a thread-like molecular aggregate in collaboration; as a result, the thickener of the present invention has an excellent thickening action, though the thickener does not contain a polymer thickener or the amount thereof is small. The thread-like molecular aggregate means an aggregate that is composed of at least the phospholipid and the biosurfactant and that has the structure of threads entangled with each other in this disclosure. The width of the thread-like molecular aggregate is, for example, 5 nm or more and 40 nm or less. It has been known that a surfactant such as CTAB:
hexadecyltrimethylammonium bromide forms a thread-like micelle and the width of the thread-like molecular aggregate according to the present invention is severalfold larger than the width of a surfactant such as CTAB. Thus, the thread-like molecular aggregate according to the present invention maintains the entangled structure even in a relatively low concentration and may exhibit an excellent thickening action and an excellent stringiness property.

The main solvent of the thickener according to the present invention is water, and may be water only or a mixed solvent of a water-miscible organic solvent and water. The water-miscible organic solvent means an organic solvent that can be unrestrictedly miscible with water, and preferably hardly has a negative effect on a human body, especially the skin of a human. An example of the water-miscible organic solvent includes ethanol and isopropanol. When the water-miscible organic solvent is used, the ratio of the water-miscible organic solvent to the total of water and the water-miscible organic solvent is preferably greater than or equal to 0.1 mass% and 20 mass% or less, more preferably 15 mass% or less, and even more preferably 10 mass% or less or 5 mass% or less.

The thickener of the present invention exhibits a thickening property as described, even though the thickener does not contain a polymer thickener or the amount thereof is small. For example, the ratios of the phospholipid and the biosurfactant are appropriately adjusted as one embodiment of the thickener according to the present invention that does not contain a polymer thickener or in which the amount of a polymer thickener is small. Specifically, a ratio of the phospholipid to 1 part by mass of the biosurfactant may be adjusted to more than 4 parts by mass and 10 parts or less by mass. When the ratios of the phospholipid and the biosurfactant are included in the range, a molecular aggregate similar to a polymer may be formed more surely and a thickening property may be exhibited similarly to a polymer thickener. The above ratio is preferably 4.5 parts or more by mass, and preferably 8 parts or less by mass, more preferably 6 parts or less by mass.

The thickener of the present invention can be produced by an ordinary method. For example, the thickener may be produced by mixing at least the phospholipid and the biosurfactant in a solvent in addition to the other component for thickening. Alternatively, the thickener of the present invention may be separately mixed in a liquid composition to be thickened for thickening the liquid composition. The thickener of the present invention may contain a solvent or may not contain a solvent in the latter embodiment.

The thickener and the thickened composition containing the present invention thickener may contain components other than the phospholipid and the biosurfactant in addition to the solvent as an optional component. An example of such other components includes a polyol having 3 or more hydroxy groups, such as glycerin. The content amount of the polyol may be appropriately adjusted and may be adjusted to, for example, 1 part or more by mass and 20 parts or less by mass to 1 part by mass of the biosurfactant. The polyol having 3 or more hydroxy groups can be used as a solvent under atmospheric temperature and atmospheric pressure, and can be used as a moisturizing agent, a water retention agent, a moistening agent, a skin protective agent, an oral hygiene agent, a fragrance or the like. Other examples of the optional component include an ultraviolet absorber, an antioxidizing agent, an emollient agent, a solubilizing agent, an anti-inflammatory agent, a moisturizing agent, an antiseptic agent, a bactericidal agent, a dye, a fragrance and a powder.

The stickiness is suppressed and the feeling when used is excellent particularly after the thickener and the thickened composition containing the present invention thickener is dried, since the thickener and the thickened composition do not contain a polymer thickener or the amount thereof is small. Specifically, the concentration of a polymer thickener in the thickener or the thickened composition containing the present invention thickener is preferably less than 1.0 mass%. The concentration may be 0 mass%. Thus, the thickened composition containing the present invention thickener is particularly useful as a topical medication such as a cosmetic and a skin topical medication, which are directly applied to the skin.

The thickener of the present invention specifically comprises a phospholipid, a biosurfactant and water, has viscosity of 30 mPa·s or more and 600 Pa·s or less, and a content amount of a polymer thickener is less than 1.0 mass%. For example, the viscosity is determined by rotating a circular cylinder or a circular disk in a solution that contains 0.1 mass% or more and 5 mass% or less of a phospholipid, 0.2 mass% or more and 10 mass% or less of a biosurfactant and water as a solvent and that does not contain a polymer thickener or in which the concentration of a polymer thickener is less than 1.0 mass%, measuring viscosity resistance torque acting on the used circular cylinder or circular disk, and converting the measured value. The viscosity is preferably 40 mPa·s or more, more preferably 50 mPa·s or more, and preferably 500 Pa·s or less.

The present application claims the benefit of the priority date of Japanese patent application No. 2020-8230 filed on January 22, 2020. All of the contents of the Japanese patent application No. 2020-8230 filed on January 22, 2020, are incorporated by reference herein.

### EXAMPLES

Hereinafter, the present invention is described in more detail with Examples. The present invention is however not restricted to the following Examples in any way, and it is possible to work the present invention according to the Examples with an additional appropriate change within the range of the above descriptions and the following descriptions. Such a changed embodiment is also included in the technical scope of the present invention.

### Example 1

Phospholipid ("L-α-dimyristoylphosphatidylcholine" manufactured by NOF CORPORATION, hereinafter abbreviated as "DMPC") (625 mg) was weighed and added into a test tube, and chloroform was added thereto to dissolve the phospholipid. Nitrogen gas was blown to the inside of the test tube with stirring the solution using a vortex mixer to form a lipid film on the wall surface of the test tube by removing chloroform. The test tube was left to stand in a desiccator for 2 days. Then, a phosphate buffer solution of pH 7.4 (5 mL) was added thereto to be hydrated at room temperature for 10 minutes. Next, the mixture was stirred using a vortex mixer for 3 minutes to prepare 200 mM DMPC liposome aqueous solution. The concentration of DMPC was measured using a kit to determine quantity of phospholipid ("Phospholipid C-Test Wako" manufactured by Wako Chemicals).

Then, sodium surfactin (hereinafter abbreviated as "SFNa") (524 mg) was weighed and added into a test tube. Phosphate buffer solution (pH 7.4) (10 mL) or water was added thereto to adjust the concentration of SFNa in the aqueous solution to be 50 mM. The above-described 200 mM DMPC liposome aqueous solution (2480 µL) and 50 mM SFNa aqueous solution (2000 µL) were mixed with 5520 µL of phosphate buffer solution (pH 7.4) or water so that the total concentration of DMPC and SFNa was adjusted to be 4 mass% or 8 mass%. The mixture was stirred using a vortex mixer for 3 minutes and then stirred at 200 rpm using a small size constant temperature incubator shaker ("Bioshaker BR-23FD" manufactured by TAITEC Corporation) all day.

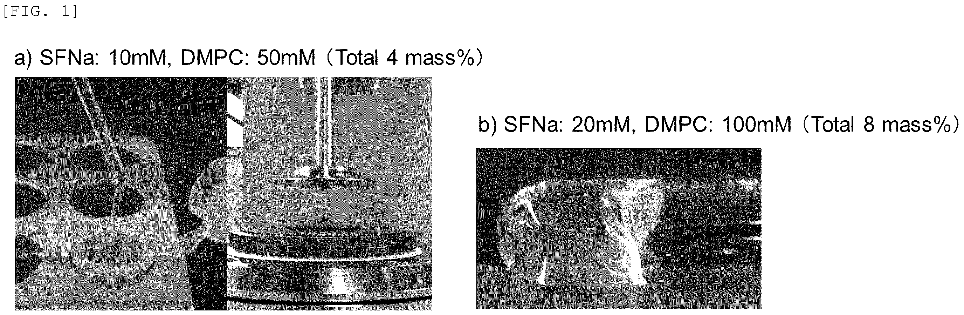

As a result, it was found as shown in Figure 1 that a viscous transparent composition can be obtained, and a thickened gelatinous composition can be obtained in the case where the ratio of DMPC is high. Even when the test tube was tilted, the surface of the thickened gelatinous composition was maintained without change. In addition, a similar result could be obtained even by weighing and adding powders of DMPC and SFNa into a test tube, adding water thereto, stirring the mixture using a vortex mixer for 1 minute, and then stirring all day as the above without preliminarily preparing a liposome, though the powder slightly remained to be undissolved. The pH of the solutions were measured; as a result, the solutions were approximately neutral and the pH was about 7.5.

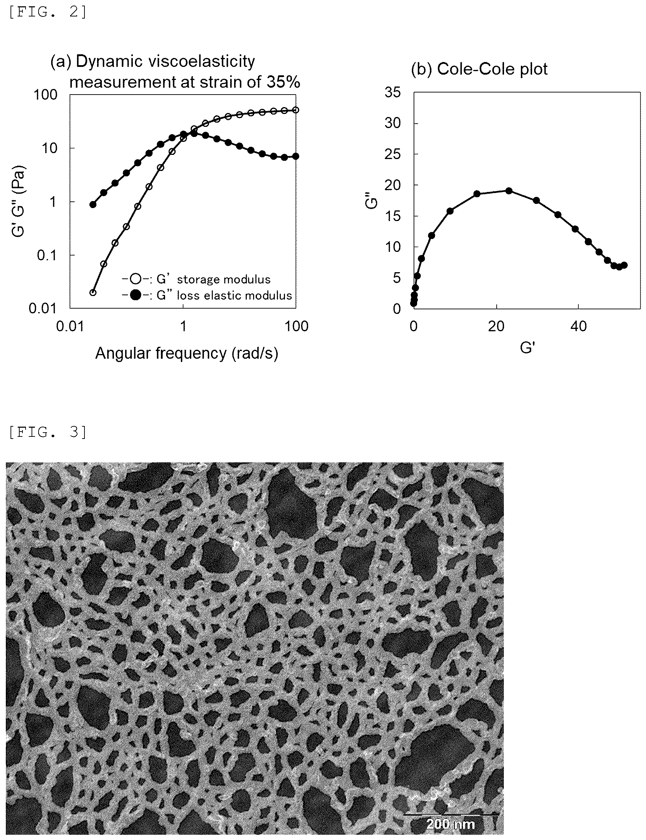

The dynamic viscoelasticity of the thickened gelatinous composition of which total ratio of DMPC and SFNa was 8 mass% was measured. The dynamic viscoelasticity was measured at 25°C using a modular compact rheometer 302 manufactured by Anton Paar and a sandblasted parallel plate having a diameter of 49.977 mm. The result is shown in Figure 2.

Figure 2(a) demonstrates the dependence of a storage modulus G' and a loss elastic modulus G" on an angular frequency in the case where the strain was fixed at 35%. It was found as a result that the gelatinous composition behaves like a typical Maxwell fluid, since the loss elastic modulus G" curve has the maximum value at the intersection with the storage modulus G' curve and the storage modulus G' curve becomes almost constant in the range of the angular frequency at the maximum of the loss elastic modulus G" curve or more.

Figure 2(b) demonstrates the Cole-Cole plot of G' vs G" of the gelatinous composition. It was found that the gelatinous composition behaves like a typical Maxwell fluid, since the data is distributed in a semicircle. Such a behavior suggests that the viscoelasticity is not derived from the entanglement of polymers and a thread-like molecular aggregate is formed in the gelatinous composition.

The composition containing a thread-like molecular aggregate according to the present invention exhibits viscosity in the static state and the fluidity thereof becomes high under applied force such as stirring. The composition containing a thread-like molecular aggregate according to the present invention exhibits a thixotropic property; therefore, the composition exhibits an excellent use property.

Specifically, dripping of the composition can be suppressed due to the viscosity by the thread-like molecular aggregate structure when the composition is taken out of a container, and the composition can be easily applied to the skin since the thread-like molecular aggregate structure is collapsed and thus the viscosity is reduced. In addition, the composition does not easily drip after being applied, since the thread-like molecular aggregate structure is constructed again after applying the composition.

When a conventional topical moisturizing formulation product is directly applied to the skin, the viscosity is decreased due to the salt on the skin. When the viscosity of the conventional product is maintained by mixing a large amount of a polymer thickener such as polysaccharide, the product has a problem of stickiness. A topical medication which maintains viscosity and which has a good feeling when used, without stickiness, can be provided by the composition of the present invention containing a thread-like molecular aggregate.

### Example 2

A gelatinous composition was prepared similarly to Example 1 except that the concentration of SFNa was changed to 10 mM and the concentration of DMPC was changed to 50 mM. The prepared gelatinous composition was rapidly frozen, and the surface was torn in a high vacuum condition. Then, after the solvent on the torn surface was removed to expose the object to be observed, a replica membrane was prepared using platinum and carbon on the surface and observed using a freeze-fracture transmission electron microscopy (FF-TEM, "JEM-1010" manufactured by JEOL). The result is shown in Figure 3.

A molecular aggregate formed by entwined threads was observed in the gelatinous composition of DMPC : SFNa = 5 : 1 by mass as Figure 3 and the average value of the thread width at optionally selected 20 parts was 20.4±3.7 nm. The thread-like molecular aggregate may be formed by stacking planar nanoparticles composed of DMPC and SFNa, since the thread width is severalfold larger than a thread-like micelle composed of a general surfactant such as CTAB.

The reason why the thickener of the present invention can increase viscosity even without a polymer may be that the thickener has the molecular aggregate structure.

## Claims

1. A thickener,
comprising a phospholipid and a biosurfactant,
wherein a ratio of the phospholipid to 1 part by mass of the biosurfactant is more than 4 parts by mass and 10 parts or less by mass.

2. The thickener according to claim 1, comprising a thread-like molecular aggregate comprising the phospholipid and the biosurfactant.

3. The thickener according to claim 2, comprising 1 part or more by mass and 20 parts or less by mass of a polyol to 1 part by mass of the biosurfactant.

4. The thickener according to any one of claims 1 to 3, wherein the biosurfactant is surfactin represented by the following formula (II) or a salt thereof: 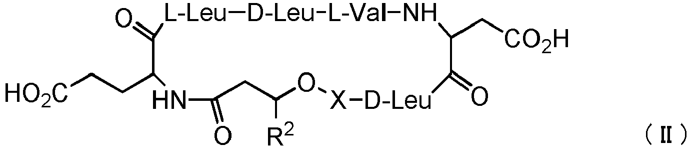 wherein X is an amino acid residue selected from leucine, isoleucine and valine, and R² is a C₉₋₁₈ alkyl group.

5. A topical medication comprising the thickener according to any one of claims 1 to 4.

6. The topical medication according to claim 5, wherein the topical medication is a cosmetic.

7. Use of a thickener for increasing viscosity of a composition comprising water, wherein the composition comprises a phospholipid and a biosurfactant, and a ratio of the phospholipid to 1 part by mass of the biosurfactant is more than 4 parts by mass and 10 parts or less by mass.

8. The use according to claim 7, wherein the thickener comprises a thread-like molecular aggregate comprising the phospholipid and the biosurfactant.

9. The use according to claim 7 or 8, wherein the thickener comprises 1 mass part or more by mass and 20 parts or less by mass of a polyol to 1 part by mass of the biosurfactant.

10. The use according to any one of claims 7 to 9, wherein the biosurfactant is surfactin represented by the following formula (II) or a salt thereof: 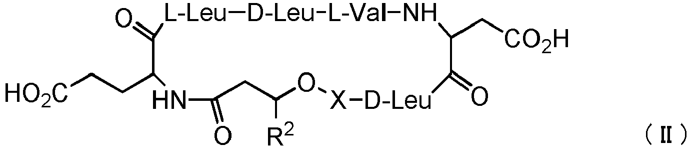 wherein X is an amino acid residue selected from leucine, isoleucine and valine, and R² is a C₉₋₁₈ alkyl group.

11. The use according to any one of claims 7 to 10, wherein a concentration of a polymer thickener in the composition is less than 1.0 mass%.

12. A method for increasing viscosity of a composition comprising water,
comprising the step of mixing a thickener in the composition,
wherein the thickener comprises a phospholipid and a biosurfactant, and a ratio of the phospholipid to 1 part by mass of the biosurfactant is more than 4 parts by mass and 10 parts or less by mass.

13. The method according to claim 11, wherein the thickener comprises a thread-like molecular aggregate comprising the phospholipid and the biosurfactant.

14. The method according to claim 12 or 13, wherein the thickener comprises 1 mass part or more by mass and 20 parts or less by mass of a polyol to 1 part by mass of the biosurfactant.

15. The method according to any one of claims 12 to 14, wherein the biosurfactant is surfactin represented by the following formula (II) or a salt thereof: 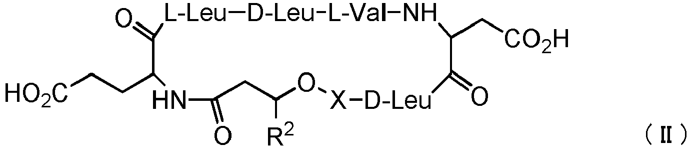 wherein X is an amino acid residue selected from leucine, isoleucine and valine, and R² is a C₉₋₁₈ alkyl group.

16. The method according to any one of claims 12 to 15, wherein a concentration of a polymer thickener in the composition is less than 1.0 mass%.
